(19) Europäisches Patentamt
European Patent Office
Office européen des brevets

(11) EP 3 890 833 B1

(12) # EUROPEAN PATENT SPECIFICATION

(45) Date of publication and mention of the grant of the patent:
**07.06.2023 Bulletin 2023/23**

(21) Application number: **19813837.2**

(22) Date of filing: **04.12.2019**

(51) International Patent Classification (IPC):
***A61P 25/00*** *(2006.01)* ***C07D 207/27*** *(2006.01)*
***A61K 31/4015*** *(2006.01)* ***A61P 25/02*** *(2006.01)*
***A61P 25/24*** *(2006.01)* ***A61P 25/28*** *(2006.01)*

(52) Cooperative Patent Classification (CPC):
**C07D 207/27; A61P 25/02; A61P 25/24; A61P 25/28**

(86) International application number:
**PCT/EP2019/083594**

(87) International publication number:
**WO 2020/115093 (11.06.2020 Gazette 2020/24)**

(54) **SYNERGISTIC COMPOSITIONS COMPRISING (R)-2-(2-OXOPYRROLIDIN-1-YL)BUTANAMIDE AND (S)-2-(2-OXOPYRROLIDIN-1-YL)BUTANAMIDE IN A NON-RACEMIC RATIO**

SYNERGISTISCHE ZUSAMMENSETZUNGEN MIT (R)-2-(2-OXOPYRROLIDIN-1-YL)BUTANAMID UND (S)-2-(2-OXOPYRROLIDIN-1-YL)BUTANAMID IN EINEM NICHT-RACEMISCHEN VERHÄLTNIS

COMPOSITIONS SYNERGIQUES COMPRENANT (R)-2-(2-OXOPYRROLIDIN-1-YL)-BUTANAMIDE ET (S)-2-(2-OXOPYRROLIDIN-1-YL)- BUTANAMIDE DANS UN RAPPORT NON RACÉMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2018 EP 18210106**

(43) Date of publication of application:
**13.10.2021 Bulletin 2021/41**

(73) Proprietor: **Metys Pharmaceuticals AG**
**4001 Basel (CH)**

(72) Inventors:
- **FARINA, Carlo**
  **20146 Milano (IT)**
- **SCHERZ, Michael**
  **4140 Oberwil (CH)**
- **GHELARDINI, Carla**
  **51100 Pistoia (IT)**
- **DI CESARE MANNELLI, Lorenzo**
  **59100 Prato (IT)**

(74) Representative: **Vossius & Partner**
**Patentanwälte Rechtsanwälte mbB**
**Siebertstraße 3**
**81675 München (DE)**

(56) References cited:
**EP-A1- 2 671 858** **WO-A1-2006/053441**
**WO-A1-2018/219977**

- **GÉRALDINE SPRINGUEL ET AL: "Innovative Chiral Resolution Using Enantiospecific Co-Crystallization in Solution", CRYSTAL GROWTH & DESIGN., vol. 12, no. 7, 18 June 2012 (2012-06-18), pages 3374-3378, XP055579905, US ISSN: 1528-7483, DOI: 10.1021/cg300307z cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HERMAN CHRISTELLE ET AL: "Solid-liquid phase diagrams for the determination of the solid state nature of both polymorphs of (RS)-2-(2-oxo-pyrrolidin-1-yl)-butyramide" , INTERNATIONAL JOURNAL OF PHARMACEUTICS, ELSEVIER, NL , vol. 437, no. 1-2 1 January 2012 (2012-01-01), pages 156-161, XP009518573, ISSN: 0378-5173, DOI: 10.1016/J.IJPHARM.2012.07.047 Retrieved from the Internet: URL:https://api.elsevier.com/content/article/PII:S0378517312007685?httpAccept=text/plain

## Description

### Field

The present invention relates to compositions comprising (R)-2-(2-oxopyrrolidin-1-yl)butanamide and (S)-2-(2-oxopyrrolidin-1-yl)butanamide in a certain range of ratios and pharmaceutically acceptable solvates or co-crystals thereof, pharmaceutical compositions comprising said compositions, their use as a medicament and the uses of the inventive compositions or pharmaceutical compositions for the treatment and/or prevention of a central nervous system disease or disorder typically and preferably selected from seizure-related disorders; preferably, the disease is epilepsy.

### Background

Glutamic acid is an excitatory neurotransmitter that is widely present in the brain. The first indication of its role as an excitatory messenger emerged in the 1950's, when it was observed that intravenous administration of glutamate induces convulsions. However, the detection of the entire glutamatergic neurotransmitter system, with biosynthetic and catabolic enzymes, cellular uptake mechanisms, intracellular storage and release systems, and its cell-surface ion channels and G protein-coupled receptors, did not take place until the 1970's and 1980's, when suitable pharmacological tools were first identified. It was in the 1990's that the newly emergent tools of molecular biology provided means for the molecular identification and classification of glutamatergic ion channels, receptors, transporters, etc.

The membrane-bound ion channels that are gated by the excitatory amino acids glutamate and glycine, and that also respond to the xenobiotic compound N-methyl-D-aspartate (NMDA), control the flow of both divalent and monovalent cations into pre- and post-synaptic neural cells (see Foster et al., Nature 1987, 329:395-396; Mayer et al., Trends in Pharmacol. Sci. 1990, 11:254-260). They are molecularly, electrophysiologically, and pharmacologically distinct from the glutamate-gated, cation-conducting ion channels that respond to the xenobiotic agents kainate or alpha-amino-3-hydroxy-5-methyl-4-isoxazolepropionic acid (AMPA); and they are similarly distinct from the family of glutamate-gated G protein-coupled receptors, the so-called metabotropic glutamate receptors.

The NMDA-preferring glutamate-gated ion channel has a hetero-tetrameric structural basis: two obligatory GluN1 units and two variable GluN2 receptor subunits encoded by the GRIN1 gene and one of four GRIN2 genes, respectively. One or both GluN2 subunits can be potentially replaced by a GluN3A or a GluN3B subunit. The GRIN1 gene product has 8 splice variants while there are 4 different GRIN2 genes (GRIN2A-D) encoding four distinct GluN2 subunits. The glycine binding site is present on the GluN1 subunit and the glutamate binding site is present on the GluN2 subunit (Paoletti P et al., Nat Rev Neurosci. 2013; 14(6):383-400).

Compounds that modulate NMDA receptor function can be useful in treatment of many neurological and psychiatric disorders including but not limited to bipolar disorder (Martucci L et al., Schizophrenia Res, 2006; 84(2-3):214-21), major depressive disorder (Li N et al., Biol Psychiatry. 2011; 69(8):754-61), treatment-resistant depression (Preskorn SH et al. J Clin Psychopharmacol. 2008; 28(6):631-7) and other mood disorders (including schizophrenia (Grimwood S et al., Neuroreport. 1999; 10(3):461-5), ante- and postpartum depression (Weickert CS et al. Molecular Psychiatry (2013) 18, 1185-1192), seasonal affective disorder, and the like; Alzheimer's disease (Hanson JE et al., Neurobiol Dis. 2015; 74:254- 62; Li S et al., J Neurosci. 2011; 31(18):6627-38) and other dementias (Orgogozo JM et al. Stroke 2002, 33: 1834-1839), Parkinson's disease (Duty S, CNS Drugs. 2012; 26(12):1017-32; Steece-Collier K et al., Exp Neurol. 2000; 163(1):239-43; Leaver KR et al. Clin Exp Pharmacol Physiol. 2008; 35(11):1388-94), Huntington's chorea (Tang TS et al., Proc Natl Acad Sci USA. 2005; 102(7):2602-7; Li L et al., J Neurophysiol. 2004; 92(5):2738-46), multiple sclerosis (Grasselli G et al., Br J Pharmacol. 2013; 168(2):502-17), cognitive impairment (Wang D et al. 2014, Expert Opin Ther Targets 2014; 18(10): 1121-30), head injury (Bullock MR et al., Ann N YAcad Sci. 1999; 890:51-8), spinal cord injury, stroke (Yang Y et al., J Neurosurg. 2003; 98(2):397-403), epilepsy (Naspolini AP et al., Epilepsy Res. 2012 Jun; 100(1-2):12-9), movement disorders (e.g. dyskinesias) (Morissette M et al., Mov Disord. 2006; 21(1):9-17), various neurodegenerative diseases (e.g. amyotrophic lateral sclerosis (Fuller PI et al., Neurosci Lett. 2006; 399(1-2):157-61) or neurodegeneration associated with bacterial or chronic infections, glaucoma (Naskar R et al. Semin Ophthalmol. 1999 Sep; 14(3):152-8 ), pain (e.g. chronic, cancer, post-operative and neuropathic pain (Wu LJ and Zhuo M, Neurotherapeutics. 2009; 6(4):693-702), diabetic neuropathy, migraine (Peeters M et al., J Pharmacol Exp Ther. 2007; 321(2):564- 72), cerebral ischemia (Yuan H et al., Neuron. 2015; 85(6):1305-18), encephalitis (Dalmau J. et al., Lancet Neurol. 2008; 7(12): 1091-8.), autism and autism spectrum disorders (Won H. et al., Nature. 2012; 486(7402):261-5), memory and learning disorders (Tang, Y. P. et al., Nature. 1999; 401(6748):63-9), obsessive compulsive disorder (Arnold PD et al., Psychiatry Res. 2009; 172(2):136-9.), attention deficit hyperactivity disorder (ADHD) (Dorval KM et al., Genes Brain Behav. 2007; 6(5):444-52), post-traumatic stress disorder (PTSD) (Haller J et al. Behav Pharmacol. 2011; 22(2):113-21; Leaderbrand K et al. Neurobiol Learn Mem. 2014; 113:35-40), tinnitus (Guitton MJ, and Dudai Y, Neural Plast.2007; 80904; Hu SS et al. 2016; 273(2): 325-332), sleep disorders (like narcolepsy or excessive daytime sleepiness, patent WO 2009/058261 A1), vertigo and nystagmus (Straube A. et al., Curr Opin Neurol. 2005; 18(1): 11-4; Starck M

et al. J Neurol. 1997 Jan; 244(1):9-16), anxiety, autoimmunological disorders like neuropsychiatric systemic lupus erythematosus (Kowal C et al. Proc. Natl. Acad. Sci. U.S.A. 2006; 103, 19854-19859) and addictive illnesses (e.g. alcohol addiction, drug addiction) (Nagy J, 2004, Curr Drug Targets CNS Neurol Disord. 2004; 3(3): 169-79.; Shen H et al., Proc Natl Acad Sci USA. 2011; 108(48):19407-12).

Recent human clinical studies have identified the NMDA-type glutamate-gated ion channel as a novel target of high interest for treatment of depression (Singh JB et al., Biol Psychiatry 2016; 80(6):424-431; Preskorn SH et al. J Clin Psychopharmacol 2008; 28(6):631-7). These studies were conducted using known NMDA-receptor antagonists ketamine and CP-101606, and they have shown significant reductions in depression rating scores in patients suffering with refractory depression. Although, the efficacy was significant, the side effects of using these NDMA receptor antagonists were troublesome.

NMDA-modulating small molecule agonist and antagonist compounds have been developed for potential therapeutic use. However, many of these are associated with very narrow therapeutic indices and undesirable side effects including hallucinations, ataxia, irrational behavior, and significant toxicity, all of which limit their effectiveness and/or safety. Further, 50% or more of patients with depression do not experience an adequate therapeutic response to known administered drugs. In most instances, 2 or more weeks of drug therapy are needed before meaningful improvement is observed, as noted in an open-label study on pharmacological treatment of depression. (Rush et al, Am. J. Psychiatry 2006, 163:1905).

A seizure is single occurrence of uncontrolled electrical activity in the brain, usually for short time span. It can cause numerous signs and symptoms like convulsions, thought disturbances, loss of consciousness, and/or other symptoms. Usually, seizures are considered a symptom of a disease. Epilepsy, for example, is a chronic disease of recurrent unprovoked seizures, and is one type of seizure disorder. In epilepsy, the convulsion is a sudden, violent irregular movement of a limb of the body caused by involuntary contraction of muscles usually associated with epilepsy and/or toxic agents.

A seizure disorder is a medical condition characterized by episodes of uncontrolled electrical activity in the brain, thus producing symptoms that include two or more seizures. Seizure-related disorders are distinguished from each other by their potential causes and their own set of symptoms due to the affected area in the brain:
Generalized seizures result in loss of consciousness. When motor symptoms are involved, for example in grand mal seizures (also called tonic-clonic with muscle jerks or spasms), signs and symptoms are evident from stiffness of muscles (tonic), relaxed muscles (atonic), muscles that cause sporadic short jerking of body or limbs (myoclonic), and repetitive shaking or jerking of the body (clonic). When non-motor or absence-like symptoms are involved in a generalized seizure, they can include staring into space, sometimes with eye blinking, and a lack of awareness of what is happening around a subject or patient.

Partial or focal seizures result in either no loss of consciousness or confusion for a few minutes. Patients are aware of a twitching or a change in sensation; they may become confused for a few minutes.

Unknown onset seizures involve unclassified symptoms and may share questionable features of generalized or focal seizures. Simple partial seizure disorders, for example, differ from person to person depending upon the part of the brain affected. Benign Rolandic epilepsy in children causes tongue twitching and may interfere with speech and cause drooling. Catamenial epilepsy refers to seizures that occur in relation to the menstrual cycle. Atonic seizures cause symptoms like falling usually not associated with loss of consciousness. Absence seizures cause a short loss of consciousness with little or no symptoms. Clonic seizures cause rhythmic jerks that involve both sides of the body simultaneously. Tonic seizures cause a stiffening of the muscles. Febrile seizures usually occur in children between 6 months and 5 years of age. Other symptoms of seizure disorders may include convulsions, eye blinking, a sudden loss of muscle tone, sudden head drops or crying out, unexpected falls to the ground, changes in tastes or smells, biting the tongue, or eye rolling. These examples illustrate the complexity of seizure disorders and their symptoms.

Drug therapy is effective for the majority of patients with seizure-related symptoms but, significant unmet medical needs and treatment challenges remain. These include: drug-resistant epilepsy, adverse reactions, drug interactions, the need for better identification of epileptic syndromes, and a lack of anti-epileptogenic agents that can prevent the development of epilepsy and its comorbidities. While many newer, second-generation anti-seizure drugs cause fewer drug interactions and are better tolerated than older agents, they have not been proven to be more effective, nor have they reduced the prevalence of drug-resistant epilepsy.

Thus, there remains an urgent and important medical need for the development of novel, orally-effective therapies for the prevention and treatment of seizure-related disorders.

The S-enantiomer of 2-(2-oxopyrrolidin-1-yl)butanamide (S-etiracetam, or levetiracetam, or L059) has been marketed as Keppra® for the treatment of partial onset, myoclonic, or tonic-clonic seizures as well as epilepsy. There are also reports on the pharmacology of racemic etiracetam, such as Sara et al. Psychopharmacology (Berl). 1980;68(3):235-41 and Van Aken et al. in Acta Anaesthesiol Belg. 1980;31 Suppl:21-8. The R-enantiomer of etiractam appears to be less potent than the S-enantiomer (Gower et al., Eur J Pharmacol. 1992 Nov 10;222(2-3):193-203); Verloes R, et al., Effects of nootropic drugs in a scopolamine-induced amnesia model in mice. Psychopharmacology (Berl).

1988;95(2):226-30.) The in vitro and in vivo potency of the individual enantiomers have been compared within single publications; and the in vivo potency of racemic etiracetam has been described separately. It appears that the S-enantiomer of etiracetam is more potent than either the R- or than the racemate. Available data seems to suggest that vesicle protein SV2A is the binding site for the antiepileptic drug levetiracetam (Lynch et al., Proc Natl Acad Sci USA. 2004 Jun 29;101(26):9861-6) and that the AMPA-type glutamate-gated ion channels may play a role in the mechanism of action of leviracetam (Carunchio et al., Epilepsia. 2007 Apr;48(4):654-62).

The R-enantiomer of 2-(2-oxopyrrolidin-1-yl)butanamide (R-etiracetam, or L060) has been claimed in EP 0 165 919 to be 10-fold more potent upon sub-cutaneous injection than racemic etiracetam in a rat model of electroshock-induced amnesia.

Dimiracetam is (*RS*)-3,6,7,7a-tetrahydro-1*H*-pyrrolo[1,2-a]imidazole-2,5-dione; it is a negative allosteric modulator of spinal NMDA-type glutamate receptors in rat spinal synaptosome preparations (Fariello RG, et al. Neuropharmacology. 2014, 81:85-94) and is orally active in rat models of neuropathic pain, depression, and cognitive impairment (Pinza M, et al. J Med Chem 1993, 36(26):4214-20). The present inventors have surprisingly found that non-racemic mixtures of *R* and *S*-dimiracetam lead to activities that are superior to the activities of the single enantiomers and the racemate. This finding is the basis for the copending application PCT/EP2018/064125, which is incorporated herein by reference in its entirety.

## Summary

It has now been surprisingly and unexpectedly found that compositions of (*R*)- and (*S*)-enantiomers of 2-(2-oxopyrrolidin-1-yl)butanamide having an enantiomeric excess (ee) of the (*S*)-enantiomer greater than or equal to 50% and lower than or equal to 67% can exhibit greater pharmacological potency, in particular in certain rat models of seizure-related disorders, than the corresponding individual enantiomers or than the racemate. These compositions of (*R*)- and (*S*)-enantiomers of 2-(2-oxopyrrolidin-1-yl)butanamide thus provide a synergistic effect that could not have been predicted based on the potency of the individual enantiomers or the racemate.

Thus, the inventive compositions with an enantiomeric excess of (S)-2-(2-oxopyrrolidin-1-yl)butanamide are much more efficient than the corresponding racemic mixture in reducing the severity and the frequency of seizures in a model of electrical stimulation-induced seizure kindling, and in peripheral neuropathic pain in the paw-pressure test after administration of sodium monoiodoacetate or in the prevention of oxaliplatin-induced peripheral neuropathic pain or in the improvement of cognitive function.

Therefore, the inventive compositions of (*S*)-2-(2-oxopyrrolidin-1-yl)butanamide represented by formulae (I) and (*R*)-2-(2-oxopyrrolidin-1-yl)butanamide represented by formula (II), respectively, having an enantiomeric excess (ee) of the (*S*)-enantiomer higher than or equal to 50% and lower than or equal to 67% are pharmacologically more effective at a given dose, as compared to either the pure enantiomers alone or to racemic mixtures of these compounds. The term racemic refers to a 1:1 by weight mixture of (*R*)- and (*S*)-enantiomers, which thus has an enantiomeric excess (ee) of 0%. Thus, the effect associated with the present invention is a synergistic effect that surprisingly results from a specific range of ratios between (S)-2-(2-oxopyrrolidin-1-yl)butanamide and (R)-2-(2-oxopyrrolidin-1-yl)butanamide represented by formulae (I) and (II), respectively.

The inventive compositions are beneficial and can be used for the treatment and/or prevention of seizure-related disorders.

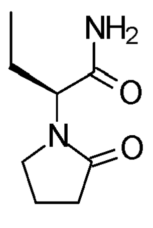

(I)

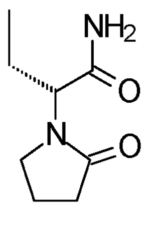

(II)

The invention is set out in the appended set of claims.

## Detailed Description

Unless defined otherwise, all technical and scientific terms used herein have the same meanings as commonly

understood by one of ordinary skill in the art to which this invention belongs.

The term "about" where used to characterize an enantiomeric excess means ±4% referring to the given numeric value, if not indicated otherwise. In each of the invention embodiments, "about" can be deleted.

The term "preferably" is used to describe features or embodiments which are not required in the present invention but may lead to improved technical effects and are thus desirable but not essential.

A number of compounds are described herein by reference to their structural formula and/or their chemical name, such as the IUPAC name. In case of discrepancies between the structural formula and the chemical name, the present invention expressly relates to the compounds as referred to by the structural formula as well as by the chemical name.

With respect to the numerical values mentioned herein, unless explicitly stated otherwise, the last decimal place of a numerical value preferably indicates its degree of accuracy. Thus, unless other error margins are given, the maximum margin is preferably ascertained by applying the rounding-off convention to the last decimal place. Thus, a value of 2.5 preferably includes the range of 2.45 to 2.54.

The term "treatment" of a disorder or disease as used herein is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease). The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, inter alia, comprise curative treatment (preferably leading to a complete response and eventually to healing of the disorder or disease) and palliative treatment (including symptomatic relief).

The term "prevention" of a disorder or disease as used herein is also well known in the art. For example, a patient/subject suspected of being prone to suffer from a disorder or disease may particularly benefit from a prevention of the disorder or disease. The subject/patient may undergo a given medical procedure known to carry the risk of developing unwanted effects, such as, for example, the development of peripheral neuropathy symptoms associated with cancer chemotherapy. The subject/patient may have a susceptibility or predisposition or risk factors for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of the aqueous pharmaceutical composition of the present invention before any clinical and/or pathological symptoms are diagnosed or determined or can be diagnosed or determined by the attending physician.

The present invention relates to compositions comprising (S)-2-(2-oxopyrrolidin-1-yl)butanamide of formula (I)) and (R)-2-(2-oxopyrrolidin-1-yl)butanamide of formula (II) in a certain ratio. It is to be understood that the term "composition" does not require that the pure compound of formula (I) and the pure compound of formula (II) have to be mixed. They can be formulated jointly or separately and be administered simultaneously or subsequently, provided that the ratio of the compound of formula (I) and the compound of formula (II) resulting in the subject to be treated is as required by the present invention. Preferably, the inventive composition is a mixture of the compound of formula (I) and the compound of formula (II), but the inventive composition may also encompass a combination of one or more articles containing the compound of formula (I) and one or more articles containing the compound of formula (II), or a combination of one or more articles containing the compound of formula (I) with one or more articles containing a mixture of the compound of formula (I) with the compound of formula (II), e.g.an about 1:1 mixture of the compound of formula (I) and the compound of formula (II).

Furthermore, 2-(2-oxopyrrolidin-1-yl)butanamide contained in the composition of the present invention has to be present in the overall range of ratios of the compound of formula (I) and the compound of formula (II), alternatively expressed as the enantiomeric excess of the compound of formula (I), required in the present invention. In other words, it is against the gist of the present invention to theoretically split a composition containing equal amounts of the compound of formula (I) and the compound of formula (II) into a component containing an excess of the compound of formula (I) and another component containing an excess of the compound of formula (II). Thus, in whichever physical form the composition of the present invention is, the composition as a whole has to fulfill the requirements regarding the range of ratios of the compound of formula (I) and the compound of formula (II), alternatively expressed as the enantiomeric

excess of the compound of formula (I), of the present invention. It is to be understood that the ratios of the compound of formula (I) and the compound of formula (II), alternatively expressed as the enantiomeric excess of the compound of formula (I), are based on a statistically meaningful number of 2-(2-oxopyrrolidin-1-yl)butanamide molecules, which typically exceeds 1000 molecules. In the present invention, the relative amounts of the compound of formula (I) and the compound of formula (II) are expressed either in terms of the ratio of the compound of formula (I) and the compound of formula (II) or in terms of the enantiomeric excess of the compound of formula (I).

It is to be understood that the "ratio" of the compound of formula (I) and the compound of formula (II) as used herein refers to the weight ratio of the compound of formula (I) and the compound of formula (II), unless explicitly stated otherwise. If solvates of the compound of formula (I) and/or the compound of formula (II) are used, the solvent is thus to be disregarded in this calculation. In other words, the "ratio of the compound of formula (I) and the compound of formula (II)" is calculated as follows:

$$\text{Ratio of the compound of formula (I) and the compound of formula (II)} = \frac{\text{amount of the compound of formula (I) by weight}}{\text{amount of the compound of formula (II) by weight}}$$

As known by the skilled person in the art, the ratio of compounds differing only in chirality, such as in the case of the compound of formula (I) and the compound of formula (II), can be determined in a number of ways known in the art, including but not limited to chromatography using a chiral support, polarimetric measurement of the rotation of polarized light, nuclear magnetic resonance spectroscopy using chiral shift reagents, or derivatization of a compound using a chiral compound such as Mosher's acid followed by chromatography or nuclear magnetic resonance spectroscopy. Enantiomers can further be isolated from mixtures by methods known to those skilled in the art, including chiral high-pressure liquid chromatography (HPLC) and direct fractional crystallization of the racemate, by chiral co-crystallization techniques, which exploit the formation of specific hydrogen bonding interactions present in co-crystals (*see* Springuel GR, *et al.,* 2012; and US Patent 6,570,036). Useful co-crystallization partners include enantiomers of mandelic acid, malic acid, tartaric acid and its derivatives; or enantiomers can be prepared by asymmetric syntheses (see, for example, Eliel and Wilen, 1994).

The ratio of the compound of formula (I) and the compound of formula (II) (which may also be referred to as the chiral purity) of the inventive composition such as the non-racemic mixture can also be expressed in terms of its enantiomeric excess (ee), typically and preferably as determined by chiral HPLC (see Examples for details), and calculated by the equation:

$$ee = (A_S - A_R)/(A_S + A_R) \times 100\%,$$

wherein As is the area of the peak of the compound of formula (I), in the HPLC chromatogram of the sample solution and $A_R$ is the area of the peak of the compound of formula (II), in the HPLC chromatogram of the sample solution.

In this respect, it is noted that, although chiral "purity" is mentioned above, the gist of the present invention is not achieving a high chiral purity of the compound of formula (I) or the compound of formula (II). Instead, the gist of the present invention is that a certain range of ratios between the compound of formula (I) or the compound of formula (II) leads to a particularly synergistic effect. As opposed to cases in which merely the purity of a compound is to be improved, i.e. where the objective is known, namely one specific compound is to be obtained in a purity of ideally 100%, the present invention is based on a previously unknown ratio of two compounds, namely the compound of formula (I) and the compound of formula (II).

The term "pharmaceutically acceptable" indicates that the compound or composition, typically and preferably the solvates, co-crystals or carrier, must be compatible chemically or toxicologically with the other ingredient(s), typically and preferably with the inventive composition, when typically and preferably used in a formulation or when typically and preferably used for treating the animal, preferably the human, therewith. Preferably, the term "pharmaceutically acceptable" indicates that the compound or composition, typically and preferably the solvates, co-crystals or carrier, must be compatible chemically and toxicologically with the other ingredient(s), typically and preferably with the inventive composition, when typically and preferably used in a formulation or when typically and preferably used for treating the animal, preferably the human, therewith. It is noted that pharmaceutical compositions can be formulated by techniques known to the person skilled in the art, such as the techniques published in "Remington: The Science and Practice of Pharmacy", Pharmaceutical Press, 22nd edition.

A "solvate" refers to an association or complex of one or more solvent molecules and either the (*S*)-enantiomer of formula (I) or the (*R*)-enantiomer of formula (II). Examples of solvents that form solvates include, but are not limited to, water, isopropanol, ethanol, methanol, dimethyl sulfoxide (DMSO), ethyl acetate, acetic acid, and ethanolamine. The term "hydrate" refers to the complex where the solvent molecule is water.

A "co-crystal" refers to a crystalline structure that contains at least two different compounds that are solid in their pure form under ambient conditions. The at least two different compounds may include the compound of formula (I) and/or the compound of formula (II) and/or any further components of the composition or excipients of the pharmaceutical composition. Co-crystals are made from neutral molecular species, and all species remain neutral after crystallization; further, typically and preferably, they are crystalline homogeneous phase materials where two or more building compounds are present in a defined stoichiometric ratio. See hereto Wang Y and Chen A, 2013; and Springuel GR, *et al.,* 2012; and US Patent 6,570,036. It to be understood that the compounds of formula (I) and/or the compounds of formula (II) may be in the form of any polymorph. A variety of co-crystals and techniques for preparing such co-crystals are described in RSC Drug Discovery, Pharmaceutical Salts and Co-crystals, published in 2012 by the Royal Society of Chemistry and edited by Johan Wouters and Luc Quéré, in particular in chapters 15 and 16. Preferred examples of the co-crystal formers are those disclosed in Table 16.1 of this reference. Even more preferred co-crystals include co-crystals of α-hydroxy acids, α-keto acids and/or α-keto amides with the compounds of formula (I) and (II) in the (*R*) to (*S*)-ratios as disclosed herein. Examples of α-hydroxy acids include atrolactic acid, benzilic acid, 4-chloromandelic acid, citric acid, 3,4-dihydroxymandelic acid, ethyl pyruvate, galacturonic acid, gluconolactone, glucuronic acid, glucuronolactone, glycolic acid, 2-hydroxybutanoic acid, 2-hydroxypentanoic acid, 2-hydroxyhexanoic acid, 2-hydroxyheptanoic acid, 2-hydroxyactanoic acid, 2-hydroxynonanoic acid, 2-hydroxydecanoic acid, 2-hydroxyundecanoic acid, 4-hydroxymandelic acid, 3-hydroxy-4-methoxymandelic acid, 4-hydroxy-3-methoxymandelic acid, α-hydroxyarachidonic acid, α-hydroxybutyric acid, α-hydroxyisobutyric acid, α-hydroxylauric acid, α-hydroxymyristic acid, α-hydroxypalmitic acid, α-hydroxystearic acid, 3-(2'-hydroxyphenyl)lactic acid, 3-(4'-hydroxyphenyl)lactic acid, lactic acid, malic acid, mandelic acid, methyllactic acid, methylpyruvate, mucic acid, α-phenylacetic acid, α-phenylpyruvic acid, pyruvic acid, saccharic acid, tartaric acid and tartronic acid. Examples of α-keto acids include 2-ketoethanoic acid (glyoxylic acid), methyl 2-ketoethanoate, 2-ketopropanoic acid (pyruvic acid), methyl 2-ketopropanoate (methyl pyruvate), ethyl 2-ketopropanoate (ethyl pyruvate), propyl 2-ketopropanoate (propyl pyruvate), 2-phenyl-2-ketoethanoic acid (benzoylformic acid), methyl 2-phenyl-2-ketoethanoate (methyl benzoylformate), ethyl 2-phenyl-2-ketoethanoate (ethyl benzoylformate), 3-phenyl-2-ketopropanoic acid (phenylpyruvic acid), methyl 3-phenyl-2-ketopropanoate (methyl phenylpyruvate), ethyl 3-phenyl-2-ketopropanoate (ethyl phenylpyruvate), 2-ketobutanoic acid, 2-ketopentanoic acid, 2-ketohexanoic acid, 2-ketoheptanoic acid, 2-ketooctanoic acid, 2-ketododecanoic acid and methyl 2-ketooctanoate. Examples of α-keto amides include any compounds obtainable by reacting any one of the above examples of α-keto acids with primary or secondary amines.

In a first aspect, the invention provides for a composition comprising a compound of formula (I) and a compound of formula (II)

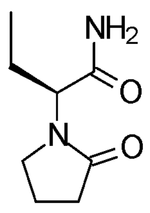

(I)

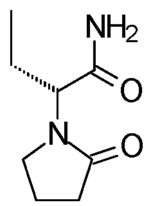

(II)

and/or pharmaceutically acceptable solvates or co-crystals thereof,

wherein the enantiomeric excess (ee) of said compound of formula (I) is equal to or higher than 50% and lower than or equal to 67%.

Typically, the non-solvated or non-co-crystallized compositions are preferred. Further preferred are the non-solvated and non-co-crystallized compositions.

In a further aspect, the invention provides for a pharmaceutical composition comprising the composition of the invention and a pharmaceutically acceptable carrier.

In again a further aspect, the invention provides for the composition of the invention or the pharmaceutical composition of the invention for use as a medicament.

In again a further aspect, the invention provides for the composition of the invention or the pharmaceutical composition of the invention for use in the treatment or prevention of seizure-related disorders. A wide variety of neurological and psychiatric symptoms and disorders may have, as their etiology, seizures or related seizure-like neurological phenomenon. In simple terms, a seizure or a related seizure-like neurological phenomenon is a single discrete clinical event caused by an excessive electrical discharge from a collection of neurons or a seizure susceptible group of neurons through a process termed "ictogenesis". As such, ictogenic seizures may be merely the symptom of a disease. However, epilepsy and other analogous seizure-related disorders are dynamic and often progressive diseases, with a maturation process characterized by a complex and poorly understood sequence of pathological transformations.

The use of the compounds and compositions of the present invention is furthermore contemplated for a psychiatric disease or disorder typically and preferably selected from depression and treatment-resistant depression, bipolar disorder, post-traumatic stress disorder, obsessive-compulsive disorder, autism spectrum disorder, schizophrenia, and anxiety; acute and chronic central sensitivity disorders such as symptoms of peripheral sensory neuropathy, preferably peripheral neuropathic pain and cold allodynia, fibromyalgia, irritable bowel syndrome, migraine, and cluster headache; and motoneuron disorders such as spinal muscular atrophy, amyotrophic lateral sclerosis, Parkinson's dystonia and Huntington's dystonia. Further examples of psychiatric diseases or disorders are set out in the following examples. The compounds and compositions of the present invention can thus also be used:

a) for the prevention or the treatment of positive symptoms of peripheral neuropathy, including cold-sensitivity, tingling, burning, or aching sensations, such as those associated with chemotherapy, antiblastic therapy, viral infection and viral treatment, post-herpetic neuralgia, osteonecrosis, trigeminal neuralgia, or diabetic peripheral neuropathy, to include the primary allodynia, secondary allodynia, or other pains or discomforts associated with sensitization of the spinal cord or higher brain structures or neuronal pathways;

b) for the prevention or the treatment of pain, including bone and joint pain, osteonecrosis pain, repetitive motion pain, dental pain, dysmenorrheal pain, cancer pain, myofascial pain, surgical pain, perioperative pain, and postsurgical pain syndromes such as post-mastectomy syndrome, post-thoracotomy syndrome, or stump pain, as well as pain associated with angina, neuroma pain, complex regional pain syndrome, chronic pelvic pain, chronic lower back pain;

c) for the prevention or the treatment of inflammatory pain, such as osteoarthritis, rheumatoid arthritis, rheumatic disease, chronic arthritic pain and related neuralgias, teno-synovitis and gout;

d) for the prevention or the treatment of neuropathic pain, such as chemotherapy-induced pain, post-traumatic injury pain, crush pain, painful traumatic mononeuropathy, painful polyneuropathy, pain resulting from spinal injury, lumbago, nerve compression or entrapment, sacral pain, trigeminal neuralgia, migraine and migraine headache, postherpetic neuralgia, phantom limb pain, post-herpetic pain, diabetic neuropathy, central pain syndrome caused a lesion at any level of the peripheral nervous system;

e) for the prevention or the treatment of neuropsychiatric disorders. Examples of neuropsychiatric disorders include schizophrenia, psychosis including schizophrenia, schizophreniform disorder, schizoaffective disorder, delusional disorder, brief psychotic disorder, substance-related disorder, paranoid schizophrenia, disorganized schizophrenia, catatonic schizophrenia or undifferentiated schizophrenia, substance- induced psychotic disorder, substance-related disorders and addictive behaviors;

f) obesity or other eating disorders associated with excessive food intake, bulimia nervosa;

g) cerebral deficits subsequent to stroke, brain edema, cerebral ischemia, cerebral hemorrhage, neurodegenerative diseases, cardiac bypass surgery and grafting, perinatal hypoxia, cardiac arrest, and hypoglycemic cerebral damage;

h) sleep disorders, such as insomnia, narcolepsy, or restless leg disorder;

i) anxiety disorders, such as affective disorder, panic attacks, panic disorder, acute stress disorder, agoraphobia, generalized anxiety disorder, obsessive-compulsive disorder, post-traumatic stress disorder, separation anxiety disorder, social phobia, specific phobia, substance-induced anxiety disorder;

j) mood disorders, such as depression, anhedonia, unipolar depression, bipolar disorder, psychotic depression;

k) substance addiction, drug dependence, tolerance, dependence or withdrawal from substances including alcohol, amphetamines, cannabis, cocaine, hallucinogens, inhalants, nicotine, opioids, phencyclidine, sedatives, hypnotics or anxiolytics;

l) impaired cognitive function, such as age related cognitive decline or cognitive disorders such as the different types of dementia associated with Alzheimer's disease, ischemia, trauma, vascular problems or stroke, HIV disease, Parkinson's disease, Huntington's disease, Pick's disease, Creutzfeldt- Jacob disease, chemotherapy, perinatal hypoxia, other general medical conditions or substance abuse;

m) Parkinson's disease, including drug-induced parkinsonism, or post-encephalitic parkinsonism;

n) attention deficit disorders, such as attention-deficit hyperactivity disorder (ADHD), obsessive-compulsive disorder, phobia, posttraumatic stress syndrome, autism and autism-spectrum disorders, impulse control disorder;

o) tinnitus, presbycusis;

p) to enhance learning and memory;

q) for the prevention or for the treatment of inherited or sporadic motor neuron disorders. Examples thereof include

amyotrophic lateral sclerosis, primary lateral sclerosis, progressive muscular atrophy, progressive bulbar palsy, Friedrich's ataxia, fragile X syndrome;

r) for the prevention or for the treatment of movement disorders. Examples thereof include dystonia, chorea, including Huntington's chorea, Parkinson's-related dystonia, Creutzfeldt-Jakob disease, progressive supranuclear palsy, multiple system atrophy, corticobasal degeneration, basal ganglia calcification;

s) for akinesias such as akinetic-rigid syndromes,

t) for dyskinesias such as medication-induced parkinsonism such as neuroleptic-induced parkinsonism, neuroleptic malignant syndrome, neuroleptic-induced acute dystonia, neuroleptic-induced acute akathisia, neuroleptic- induced tardive dyskinesia and medication-induced postural tremor, including rest tremor, postural tremor and intention tremor, chorea (such as Sydenham's chorea, Huntington's disease, benign hereditary chorea, neuroacanthocytosis, symptomatic chorea, drug-induced chorea and hemiballism), generalized or focal myoclonus, tics (including simple tics, complex tics and symptomatic tics), and dystonia (including generalised dystonia such as iodiopathic dystonia, drug-induced dystonia, symptomatic dystonia and paroxymal dystonia, and focal dystonia such as blepharospasm, oromandibular dystonia, spasmodic dysphonia, spasmodic torticollis, axial dystonia, dystonic writer's cramp and hemiplegic dystonia), muscular spasms and disorders associated with muscular spasticity or weakness including tremors;

u) and for urinary incontinence, multiple system atrophy, tuberous sclerosis, olivo-ponto-cerebellar atrophy, cerebral palsy, drug-induced optic neuritis, ischemic retinopathy, diabetic retinopathy, glaucoma, spasticity, myoclonus, and Tourette's syndrome-associated dyskinesias.

Furthermore, the compositions of the present invention can also be used to enhance learning and memory in healthy subjects, e.g. in the form of a non-therapeutic use.

It is furthermore preferred that the composition or the pharmaceutical composition is administered in association with at least one anti-seizure drug, wherein said associated administration of said composition or said pharmaceutical composition with said at least one anti-seizure drug is concurrent, simultaneous, sequential or separate.

Non-limiting examples of such anti-seizure drugs are selected from the group consisting of eslicarbazepine, lacosamide, rufinamide, pregabalin, stiripentol, oxcarbazepine, tiagabine, fosphenytoin, topiramate, gabapentin, felbamate, lamotrigine, zonisamide and vigabatrin.

The composition or the pharmaceutical composition is preferably used with at least one anti-seizure drug, wherein preferably said anti-seizure drug is selected from eslicarbazepine, lacosamide, rufinamide, pregabalin, stiripentol, oxcarbazepine, tiagabine, fosphenytoin, topiramate, gabapentin, felbamate, lamotrigine, zonisamide and vigabatrin.

Said disease or disorder is preferably seizure. The dosage will depend on the route of administration, the severity of the disease, age and weight of the patient or subject and other factors normally considered by the attending physician, when determining the individual regimen and dosage level for a particular patient or subject.

The composition or pharmaceutical composition of the invention may be administered via any route, including oral, intramuscular, subcutaneous, topical, transdermal, intranasal, intravenous, sublingual or intrarectal administration. Typically and preferably, the pharmaceutical composition of the invention is administered in a single dosage unit once-daily, twice-daily or three times-daily via the oral route, and most preferably once-daily or twice-daily. In the most preferred embodiment, the composition or pharmaceutical composition of the invention is administered twice daily.

Typically and preferably, the oral dose of the inventive composition or the inventive pharmaceutical composition is between 10 mg and 3000 mg per administration, more preferably between 20 mg to 2000 mg per administration, again more preferably between 50 mg and 1000 mg per administration. Typically and preferably, said composition or said pharmaceutical composition is administered orally twice daily in a dose of between 10 mg and 3000 mg per administration, more preferably between 20 mg to 2000 mg per administration, again more preferably between 50 mg and 1000 mg per administration.

The pharmaceutical composition of the invention may be prepared by mixing suitably selected and pharmaceutically acceptable excipients, vehicles, adjuvants, additives, surfactants, desiccants or diluents known to those well-skilled in the art, and can be suitably adapted for oral, parenteral or topical administration. Typically and preferably the pharmaceutical composition of the invention is administered in the form of a tablet, capsule, sachets, powder, granule, pellet, oral or parenteral solution, suspension, suppository, ointment, cream, lotion, gel, paste and/or may contain liposomes, micelles and/or microspheres.

The pharmaceutically acceptable carrier of the pharmaceutical composition of the invention is without limitation any pharmaceutically acceptable excipient, vehicle, adjuvant, additive, surfactant, desiccant or diluent. Suitable pharmaceutically acceptable carriers are magnesium carbonate, magnesium stearate, talc, lactose, sugar, pectin, dextrin, starch, tragacanth, methyl cellulose, sodium carboxymethyl cellulose, a low-melting wax, cocoa butter. Pharmaceutically acceptable carriers of the invention can be solid, semi-solid or liquid.

Tablets, capsules or sachets for oral administration are usually supplied in dosage units and may contain

conventional excipients, such as binders, fillers, diluents, tableting agents, lubricants, detergents, disintegrants, colorants, flavors and wetting agents. Tablets may be coated in accordance to methods well known in the art. Suitable fillers include or are preferably cellulose, mannitol, lactose and similar agents. Suitable disintegrants include or are preferably starch, polyvinyl pyrrolidone and starch derivatives such as sodium starch glycolate. Suitable lubricants include or are preferably, for example, magnesium stearate. Suitable wetting agents include or are preferably sodium lauryl sulfate. These solid oral compositions can be prepared with conventional mixing, filling or tableting methods. The mixing operations can be repeated to disperse the active agent in compositions containing large quantities of fillers. These operations are conventional.

The oral liquid compositions can be provided in the form of, for example, aqueous solutions, emulsions, syrups or elixirs or in the form of a dry product to be reconstituted with water or with a suitable liquid carrier at the time of use. The liquid compositions can contain conventional additives, such as suspending agents, for example sorbitol, syrup, methylcellulose, gelatin, hydroxyethyl cellulose, carboxymethyl cellulose, aluminum stearate gel or hydrogenated edible fats; emulsifying agents, for example lecithin, sorbitan monooleate, or acacia; non aqueous carriers (which can include edible oil), for example almond oil, fractionated coconut oil, oily esters, such as glycerin esters, propylene glycol or ethyl alcohol; preservatives, for example methyl or propyl p-hydroxybenzoate or sorbic acid and if desired, conventional flavors or colorants. Oral formulations may also include or may be formulated as conventional formulations, such as tablets or granules. For parenteral administration, liquid dosage units can be prepared containing the inventive composition and a sterile carrier.

Oral formulations may optionally further include taste-masking components to optimize the taste perception of the oral formulation. Examples of such taste-masking components may be citrus-, licorice-, mint-, grape-, black currant- or eucalyptus-based flavorants known to those well-skilled in the art.

The parenteral solutions are normally prepared by dissolving the compound in a carrier and sterilizing by filtration, before filling suitable vials or ampoules and sealing.

Adjuvants, such as local anesthetics, preservatives and buffering agents can be added to the pharmaceutical composition. In order to increase stability, the composition can be frozen after filling the vial and the water removed under vacuum. A surfactant or humectant can be advantageously included in the pharmaceutical composition in order to facilitate uniform distribution of the inventive composition.

Topical formulations include or are preferably ointments, creams, lotions, gels, gums, solutions, pastes or may contain liposomes, micelles or microspheres.

Subjects to be treated by the composition or pharmaceutical composition of the invention are humans and animals. Preferred animals are domestic and farm animals, including but not limited to guinea pig, rabbit, horse, donkey, camel, cow, sheep, goat, pig, cat, dog and parrot. More preferred subjects are mammals, again more preferably humans.

Not encompassed by the invention is an article of manufacture comprising the composition of the invention or the pharmaceutical composition of the invention, a container or package and a written description and administration instruction such as a package insert.

It is further envisaged that compositions of the compound of formula (I), or the compound of formula (II) with racetams such as aniracetam, brivaracetam, cebaracetam, coluracetam, dimiracetam, doliracetam, dupracetam, fasoracetam, imuracetam, methylphenylpiracetam, nebracetam, nefiracetam, omberacetam (Noopept), oxiracetam, phenylpiracetam, phenylpiracetam hydrazide, piracetam, pramiracetam, rolipram, rolziracetam and/or seletracetam may also be used to prepare synergistic mixtures and compositions, in particular if the ratio of the compound of formula (I), or the compound of formula (II), and the racetam, or an enantiomer of the other racetam, are chosen within the ranges disclosed herein for the mixtures of the compound of formula (I) and the compound of formula (II).

It is further envisaged that compositions of the compound of formula (I), or the compound of formula (II) with other compounds, such as those disclosed in US 7,544,705 or in US 8,334,286, may also be used to prepare synergistic mixtures and compositions, in particular if the ratio of the compound of formula (I) or the compound of formula (II), and the dimiracetam-like compound or an enantiomer of a dimiracetam-like compound, are chosen within the ranges disclosed herein for the mixtures of the compound of formula (I), and the compound of formula (II).

The non-patent references cited herein are abbreviated by first author accompanied by the year of publication. The complete citations are listed in the following.

Attal N, Cruccu G, Baron R, Haanpää M, Hansson P, Jensen TS, Nurmikko T; European Federation of Neurological Societies. EFNS guidelines on the pharmacological treatment of neuropathic pain: 2010 revision. Eur J Neurol. 2010 Sep;17(9):1113-e88. PMID: 20402746

Bradford MM. A rapid and sensitive method for the quantitation of microgram quantities of protein utilizing the principle of protein-dye binding. Anal Biochem. 1976 May 7;72:248-54. PMID: 942051

Bril V, England J, Franklin GM, Backonja M, Cohen J, Del Toro D, Feldman E, Iverson DJ, Perkins B, Russell JW, Zochodne D; American Academy of Neurology; American Association of Neuromuscular and Electrodiagnostic Medicine; American Academy of Physical Medicine and Rehabilitation. Evidence-based guideline: Treatment of

painful diabetic neuropathy: report of the American Academy of Neurology, the American Association of Neuromuscular and Electrodiagnostic Medicine, and the American Academy of Physical Medicine and Rehabilitation. Neurology. 2011 May 17;76(20):1758-65. Erratum in: Neurology. 2011 Aug 9;77(6):603. PMID: 21482920

Camilleri P, Eggleston D, Farina C, Murphy JA, Pfeiffer U, Pinza M, Senior LA. Chiral high-performance liquid chromatography of some related bicyclic lactams. Journal of Chromatography A, 654 (1993) 207-213.

Cavaletti G, Tredici G, Petruccioli MG, Dondè E, Tredici P, Marmiroli P, Minoia C, Ronchi A, Bayssas M, Etienne GG. Effects of different schedules of oxaliplatin treatment on the peripheral nervous system of the rat. Eur J Cancer. 2001 Dec;37(18):2457-63. PMID: 11720843

Christensen D, Idänpään-Heikkilä JJ, Guilbaud G, Kayser V. The antinociceptive effect of combined systemic administration of morphine and the glycine/NMDA receptor antagonist, (+)-HA966 in a rat model of peripheral neuropathy. Br J Pharmacol. 1998 Dec;125(8):1641-50. Erratum in: Br J Pharmacol 1999 Apr;126(8):1881. PMID: 9886755

Dubinsky RM, Kabbani H, El-Chami Z, Boutwell C, Ali H; Quality Standards Subcommittee of the American Academy of Neurology. Practice parameter: treatment of postherpetic neuralgia: an evidence-based report of the Quality Standards Subcommittee of the American Academy of Neurology. Neurology. 2004 Sep 28;63(6):959-65. PMID: 15452284

Di Cesare Mannelli L, Maresca M, Farina C, Scherz MW, Ghelardini C. A model of neuropathic pain induced by sorafenib in the rat: Effect of unifiram. Neurotoxicology. 2015a Sep;50:101-7. PMID: 26254739

Di Cesare Mannelli L, Micheli L, Farina C, Scherz M, and Ghelardini C. Effects of unifiram on oxaliplatin-induced hyperalgesia and allodynia in the rat. Journal of Clinical Oncology 2015b 33:15_suppl, e20650-e20650.

Eliel EL, Wilen SH. Stereochemistry of Organic Compounds, Wiley-Interscience, New York (1994). ISBN: 978-0-471-01670-0

Farina C, Gagliardi S, Ghelardini C, Martinelli M, Norcini M, Parini C, Petrillo P, Ronzoni S. Bioorg Med Chem. 2008; 16(6):3224-32. PMID: 18171618

Fariello RG, Ghelardini C, Di Cesare Mannelli L, Bonanno G, Pittaluga A, Milanese M, Misiano P, Farina C. Broad spectrum and prolonged efficacy of unifiram in models of neuropathic pain. Neuropharmacology. 2014 Jun;81:85-94. PMID: 24486381

Fariello RG, Ghelardini C, Di Cesare Manneli L, Zanardelli M, Farina C. Antidepressant-like activity of unifiram (NT-11624) in the rat forced swimming test. Program No. 789.08/EE25. 2011 Neuroscience Meeting Planner. Washington, DC: Society for Neuroscience, 2011. Online.

Fernihough J, Gentry C, Malcangio M, Fox A, Rediske J, Pellas T, Kidd B, Bevan S, Winter J. Pain related behaviour in two models of osteoarthritis in the rat knee. Pain. 2004; Nov;112(1-2):83-93. PMID: 15494188

Finnerup NB, Attal N, Haroutounian S, McNicol E, Baron R, Dworkin RH, Gilron I, Haanpää M, Hansson P, Jensen TS, Kamerman PR, Lund K, Moore A, Raja SN, Rice AS, Rowbotham M, Sena E, Siddall P, Smith BH, Wallace M. Pharmacotherapy for neuropathic pain in adults: a systematic review and meta-analysis. Lancet Neurol. 2015 Feb;14(2):162-73. PMID: 25575710

Gronseth G, Cruccu G, Alksne J, Argoff C, Brainin M, Burchiel K, Nurmikko T, Zakrzewska JM. Practice parameter: the diagnostic evaluation and treatment of trigeminal neuralgia (an evidence-based review): report of the Quality Standards Subcommittee of the American Academy of Neurology and the European Federation of Neurological Societies. Neurology. 2008 Oct 7;71(15):1183-90. PMID: 18716236

Guingamp C, Gegout-Pottie P, Philippe L, Terlain B, Netter P, Gillet P. Mono-iodoacetate-induced experimental osteoarthritis: a dose-response study of loss of mobility, morphology, and biochemistry. Arthritis Rheum. 1997 Sep;40(9): 1670-9. PMID: 9324022

Guzman RE, Evans MG, Bove S, Morenko B, Kilgore K. Mono-iodoacetate-induced histologic changes in subchondral bone and articular cartilage of rat femorotibial joints: an animal model of osteoarthritis. Toxicol Pathol. 2003;31(6):619-24. PMID: 14585729

Latremoliere A, Woolf CJ. Central sensitization: a generator of pain hypersensitivity by central neural plasticity. J Pain. 2009 Sep;10(9):895-926. PMID: 19712899.

Hansen KB, Yi F, Perszyk E, Furukawa H, Wollmuth LP, Gibb AJ, Traynelis SF. Structure, function, and allosteric modulation of NMDA receptors. J Gen Physiol 2018, 150:8, 1081-1105.

Manetti D, Ghelardini C, Bartolini et al. Design, synthesis and preliminary pharmacological evaluation of 1,4-diazabicyclo[4.3.0]nonan-9-ones as a new class of highly potent nootropic drugs. J Med Chem 2000;43:1969-1974.

Martini E, Ghelardini C, Bertucci C, et al. Enantioselective synthesis and preliminary pharmacological evaluation of the enantiomers of unifiram (DM232), a potent cognition-enhancing agent. Med Chem 2005;1:473-480.

Marieb EN, Wilhelm PB & Mallat JB, eds. Human Anatomy, 8th edition. Pearson, London (2017).

Moulin D, Boulanger A, Clark AJ, Clarke H, Dao T, Finley GA, Furlan A, Gilron I, Gordon A, Morley-Forster PK, Sessle BJ, Squire P, Stinson J, Taenzer P, Velly A, Ware MA, Weinberg EL, Williamson OD; Canadian Pain Society. Pharmacological management of chronic neuropathic pain: revised consensus statement from the Canadian Pain Society. Pain Res Manag. 2014Nov-Dec;19(6):328-35. PMID: 25479151

Nakamura Y, Iga K, Shibata T, Shudo M, Kataoka K. Glial plasmalemmal vesicles: a subcellular fraction from rat hippocampal homogenate distinct from synaptosomes. Glia. 1993 Sep;9(1):48-56. PMID: 7902337

Paluzzi S, Alloisio S, Zappettini S, Milanese M, Raiteri L, Nobile M, Bonanno G. Adult astroglia is competent for Na+/Ca2+ exchanger-operated exocytotic glutamate release triggered by mild depolarization. J Neurochem. 2007 Nov;103(3): 1196-207. PMID: 17935604

Pinza M, Farina C, Cerri A, Pfeiffer U, Riccaboni MT, Banfi S, Biagetti R, Pozzi O, Magnani M, Dorigotti L. Synthesis and pharmacological activity of a series of dihydro-1H-pyrrolo[1,2-a]imidazole-2,5(3H,6H)-diones, a novel class of potent cognition enhancers. J Med Chem. 1993 Dec 24;36(26):4214-20. PMID: 8277504

Randall LO, Selito JJ. A method for measurement of analgesic activity on inflamed tissue. Arch Int Pharmacodyn Ther. 1957 Sep 1;111(4):409-19. PMID: 13471093

Scapecchi S, Martini E, Manetti D, et al. Structure-activity relationship studies on unifiram (DM232) and sunifiram (DM235), two novel and potent cognition enhancing drugs. Bioorg Med Chem 2004;12:71-85.

Smith BH, Torrance N, Bennett MI, Lee AJ. Health and quality of life associated with chronic pain of predominantly neuropathic origin in the community. Clin J Pain. 2007 Feb;23(2):143-9. PMID: 17237663

Springuel GR, Leyssens T. Innovative chiral resolution using enantiospecific co-crystallization in solution. Cryst Growth Des. 2012; 12 (7): 3374-3378. DOI: 10.1021/ cg300307z.

Torchio L, Lombardi F, Visconti M, Doyle E. Determination of the polar drug unifiram in human plasma and serum by column-switching high-performance liquid chromatography. J Chromatogr B Biomed Appl. 1995 Apr 7;666(1):169-77. PMID: 7655615

van Hecke O, Austin SK, Khan RA, Smith BH, Torrance N. Neuropathic pain in the general population: a systematic review of epidemiological studies. Pain. 2014 Apr;155(4):654-62. Erratum in: Pain. 2014 Sep;155(9):1907. PMID: 24291734

Wang Y, Chen A. Crystallization-based separation of enantiomers, in Stereoselective Synthesis of Drugs and Natural Products, 2nd volume; Andrushko V, Andrushko N, Eds. Wiley-Interscience, New York (2013). ISBN: 978-1-118-03217-6

Zilliox LA. Neuropathic Pain. Continuum (Minneap Minn). 2017 Apr;23(2, Selected Topics in Outpatient Neurology):512-532. PMID: 28375916.

## EXAMPLES

Examples of the present invention are purely for illustrative and non-limiting purposes. Samples of racemic 2-(2-oxopyrrolidin-1-yl)butanamide as well as the individual enantiomers of formulae (I) and (II) can be synthesized using commercially available starting materials, such as purchased from Sigma-Aldrich. These commercial supplies can be used as received from the supplier without further purification, using methods and techniques of preparative synthesis well known to those skilled in the art.

### EXAMPLE 1: Synthesis of 2-(2-oxopyrrolidin-1-yl)butanamide

(*R*)-, (*S*)-and racemic mixture of 2-(2-oxopyrrolidin-1-yl)butanamide were prepared in accordance with methods described in EP 0 165 919 and in US 6,784,197. The enantiomeric excess of (*R*)- and (*S*)-derivatives, when used separately for preparing the composition of the present invention, was equal to or greater than 96% for each enantiomer.

For achieving the desired enantiomeric excess of equal to or higher than 20% ee (excess (*S*)) and less than or equal to 50% ee (excess (*S*)), as well as other desired specific compositions in accordance with the present invention, several methods known to the skilled person in the art can be applied. For example, said compositions were prepared either by mixing the individual enantiomers or by mixing the racemate of 2-(2-oxopyrrolidin-1-yl)butanamide with the respective quantities of the (*S*)-enantiomer. Furthermore, starting from a racemic 2-(2-oxopyrrolidin-1-yl)butanamide, part or all of the (*R*)-enantiomer can be removed by preparative chiral column chromatography.

### EXAMPLE 2: Mouse model of pentylenetetrazole-induced seizures

By this experiment, the dose-response relationship of the protective effect of levetiracetam in the published mouse model ((Gower et al. (Eur. J. Pharmacol. 222, 1992, 193-203)) of tonic and clonic convulsions kindled by once-daily pentylenetetrazole (PTZ) injections was assessed. Furthermore, the protective effect of levetiracetam was compared with that exhibited by racemic etiracetam or by two mixtures of levetiracetam and etiracetam, at a fixed dose. The two mixtures tested differed in the ratio of the R enantiomer of etiracetam in the mixture (i.e. 1-to-3 R-to-S-etiracetam, and 1-to-5 R-to-S-etiracetam).

**Animals**

Adult male CD1 mice (Envigo, Italy 20-25 g weight at arrival), were used. The cages were brought into the experimental room the day before the experiment, for acclimatization purposes. Each treatment group consisted of 15 mice. Mice were fed a standard laboratory diet. Food and water were freely available. Room temperature and relative humidity were constantly kept at 22 ± 2°C and 55 ± 15%, respectively, and a 12-hour light and 12-hour darkness cycles maintained.

**Drugs**

Levetiracetam (batch P102-14050 supplied by Astatech), etiracetam (supplied by Shaanxi Swiden Biotech Co., Ltd. Catalog number E932970), or mixtures of 1 part levetiracetam and 1 part etiracetam (1-to-3 R-to-S-etiracetam), or of 1 part levetiracetam and 0.5 part etiracetam (1-to-5 R-to-S-etiracetam), were dissolved in 1% carboxymethylcellulose (CMC) solution at a formulation strength of 55 mg/mL and orally administered once per day. Compounds were administered every day, from day 1 to day 11, 60 min before PTZ (55 mg/kg i.p.). PTZ was dissolved in saline; following i.p. injection, seizures developed in vehicle-treated mice within about 20 minutes.

**PTZ-induce kindling test**

The test was performed according to Gower et al. (Eur. J. Pharmacol. 222, 1992, 193-203). Separate groups of male mice were administered orally once daily for 11 consecutive days with levetiracetam (10, 30, or 60 mg/kg p.o.); etiracetam (30 mg/kg p.o.); or 1-to-3 R-to-S-etiracetam (30 mg/kg p.o.); or 1-to-5 R-to-S-etiracetam, (30 mg/kg p.o.); or oral vehicle, 60 min prior to administration of PTZ (pentylenetetrazole, 55.0 mg/kg i.p.). Immediately after the PTZ injection, the mice were placed in individual cages and observed for tonic and clonic seizures.

**Statistical analysis**

A chi-square test was used to verify significance between two means of behavioral results. Data were analyzed by the Social Science Statistics Program. P values ≤ 0.05 were considered significant (link https://www.socscistatistics.com/tests/chisquare/default2.aspx).

**Results**

The results are summarized in the following Tables 1 and 2:

***Table 1:*** *Dose response curve of levetiracetam on the development of PTZ- induced kindling in mice. Groups of 15 mice were dosed once daily with either vehicle or with levetiracetam (10-30-60 mg/kg p.o.) 60 min before PTZ was administered (55 mg/kg i.p.). Each value is the percentage of mice per group with clonic convulsions. # P< 0.05 vs PTZ treated mice.*

| *TREATMENT* | Day 1 | Day 2 | Day 3 | Day 4 | Day 5 | Day 6 | Day 7 | Day 8 | Day 9 | Day 10 | Day 11 |
|---|---|---|---|---|---|---|---|---|---|---|---|
| vehicle + PTZ | 20 | 26.6 | 26.7 | 33.3 | 33.3 | 46.6 | 53.3 | 66.6 | 86.6 | 93.3 | 100 |
| levetiracetam 10 mg/kg p.o. + PTZ | 6.6 | 6.6 | 20.0 | 26.6 | 26.6 | 53.0 | 53.3 | 73.3 | 86.6 | 86.6 | 100 |
| levetiracetam 30 mg/kg p.o. + PTZ | 0 | 6.6 | 13.3 | 20.0 | 20.0 | 26.6 | 33.3 | 46.6 | 66.6 | 66.6 | 73.3 |
| levetiracetam 60 mg/kg p.o. + PTZ | 0 | 0 | 6.6 | 20.0 | 20.0 | 20.0 | 26.6 | 26.6# | 33.3# | 33.3# | 40.0# |

***Table 2:*** *Effect of levetiracetam in comparison with etiracetam and two mixture of levetiracetam and etiracetam (respectively 1 part and 1 part and 1 part and 0.5 part) on the development of PTZ- induced kindling in mice. Groups of 15 mice were dosed once daily with either vehicle or with the test compound 60 min before PTZ was administered (55 mg/kg i.p.). Each value is the percentage of mice per group with clonic convulsions. * P< 0.05 vs levetiracetam -treated mice.*

| *TREATMENTS* | *Day 1* | *Day 2* | *Day 3* | *Day 4* | *Day 5* | *Day 6* | *Day 7* | *Day 8* | *Day 9* | *Day 10* | *Day 11* |
|---|---|---|---|---|---|---|---|---|---|---|---|
| PTZ | 13.3 | 13.3 | 20.0 | 26.6 | 33.3 | 46.6 | 53.3 | 80.0 | 86.6 | 100 | 100 |
| levetiracetam 30 mg/kg p.o. | 0 | 0 | 6.6 | 13.3 | 20.0 | 26.6 | 33.3 | 46.6 | 66.6 | 73.3 | 73.3 |
| etiracetam 30 mg/kg p.o. | 0 | 6.6 | 13.3 | 26.6 | 26.6 | 46.6 | 53.3 | 66.6 | 86.6 | 93.3 | 100 |
| Mixture of levetiracetam (1 part) and etiracetam (1 part) 3:1 S:R 30 mg/kg p.o. | 0 | 0 | 0 | 6.6 | 13.6 | 13.6 | 13.6 | 13.6* | 20.0* | 26.6* | 33.3* |
| Mixture of levetiracetam (1 part) and etiracetam (0.5 part) 5:1 S:R 30 mg /kg p.o. | 0 | 0 | 6.6 | 6.6 | 13.6 | 13.6 | 20.0 | 33.3 | 40.0 | 46.6 | 53.3 |

Single daily injections of PTZ (55 mg/kg i.p) produced a progressive increase in the proportion of vehicle-control-treated mice with tonic or clonic convulsions, increasing from less than 25% on day 1 to 100% on day 11 (Tab. 1). Pretreatment with levetiracetam (10, 30 or 60 mg/kg p.o.) caused a dose-dependent reduction of this phenomenon. A statistically significant difference in the proportion of mice exhibiting seizures, compared to the vehicle treatment group, is reached only at the dose of 60 mg/kg p.o. from the eighth to the eleventh day of treatment (Tab. 1). The intensity of the effect of levetiracetam was such that after 60 mg/kg p.o. the incidence of clonic convulsion did not exceed 20% in the first 6 days and reached a maximum value of 40% after 11 days of kindling. At the lower tested dose of levetiracetam (10 mg/kg p.o.), at the end of the treatment, all mice showed tonic or clonic convulsions; at the dose of 30 mg/kg p.o. an intermediate reduction of the effect was observed (Tab. 1).

In a subsequent experimental sequence, the antiepileptic effect of levetiracetam at 30 mg/kg p.o. was compared with that of racemic etiracetam (30 mg/kg p.o.) and the 1-to-3 R-to-S etiracetam mixture and the 1-to-5 S-to-R etiracetam mixture (Tab. 2).

1-to-3 R-to-S-etiracetam at 30 mg/kg p.o. was more effective than levetiracetam: the proportion of mice showing tonic/clonic convulsions was smaller than that of the levetiracetam group, and this effect was statistically significant starting from day 8 until day 11. With 1-to-5 R-to-S-etiracetam, the proportion of mice exhibiting tonic or clonic seizures on day 11 was 67 % (Tab. 2) and numerically smaller than that of the levetiracetam-treated group.

**Conclusion**

The PTZ-kindled mouse seizure model shows a dose-responsive effect of oral levetiracetam, which is consistent with previously published data. When compared at 30 mg/kg p.o., levetiracetam was moderately effective, and racemic etiracetam at 30 mg/kg p.o. was ineffective. Remarkably, the mixtures of 1-to-3 R-to-S-etiracetam and 1-to-5 R-to-S-etiracetam were more effective than levetiracetam itself; this difference achieved statistical significance from levetiracetam at the latest on days 8 to 11 for the 1-to-3 R-to-S-etiracetam mixture.

**EXAMPLE 3: Effect of the novel compositions in a model of seizures**

The anti-seizure effects of the incident novel compositions are assessed in the kindling model of temporal lobe epilepsy as described in Loscher W et al., J. Pharm. Exp. Ther., 284:474-479, 1998.

Electrode implantation. For the kindling experiments, the rats are anesthetized with chloral hydrate (360 mg/kg i.p.) and receive stereotaxic implantation, according to the surgery methods described in the atlas of Paxinos and Watson

(1986), of one bipolar electrode in the right basolateral amygdala. Coordinates for electrode implantation are AP -2.2, L -4.8, V -8.5. All coordinates are measured from the bregma. Skull screws serve as the indifferent reference electrode. The electrode assembly is attached to the skull by dental acrylic cement.

Experiments on kindling development. Four groups of eight rats each are implanted with kindling electrodes as described above. Two weeks after implantation, the initial (prekindling) electrical susceptibility of the stimulated region (ADT) is determined in each rat using an ascending stairstep procedure. The initial current intensity is 10 μA, and the current intensity is increased in steps of about 20% of the previous current at intervals of 1 min until an afterdischarge of at least 3-sec duration is elicited. On the morning of the next day, chronic treatment is started, and the animals are stimulated with a suprathreshold current of 500 μA 1 hour after each i.p. injection of the test compound. Group I receives i.p. injections of vehicle (saline), group II i.p. injections of 13 mg/kg levetiracetam, group III i.p. injections of 27 mg/kg levetiracetam and group IV i.p. injections of 54 mg/kg levetiracetam. Vehicle or drug are injected once daily 5 days a week (except on weekends). After 21 injections, treatment and amygdala stimulations are terminated. After a wash-out period of 5 days, further amygdala stimulations with 500 μA are carried out until all animals exhibit 10 stage 5 seizures. Seizure severity, seizure duration and afterdischarge duration are recorded after each stimulation, as follows. Seizure severity is classified according to Racine (1972): 1, immobility, eye closure, twitching of vibrissae, sniffing, facial clonus; 2, head nodding associated with more severe facial clonus; 3, clonus of one forelimb; 4, rearing, often accompanied by bilateral forelimb clonus; 5, rearing with loss of balance and falling accompanied by generalized clonic seizures. Seizure duration is the duration of limbic (stage 1-2) and/or motor seizures (stage 3-5); limbic seizure activity (immobility associated with low amplitude afterdischarges and occasional facial clonus or head nodding) often occurring after termination of motor seizures is not included in seizure duration. Afterdischarge duration is the total duration of amygdala electroencephalogram spikes with an amplitude of at least twice the amplitude of the prestimulus recording and a frequency greater than 1/sec.

One week after the last stage 5 seizure, the post-kindling ADT is determined in each group as described above for the prekindling ADT.

Based on the results of experiments from Examples 1 and 2, it will be possible to see that the effects of the claimed ratios of *S*:*R* enantiomers of 2-(2-oxopyrrolidin-1-yl)butanamide lead to increased anti-seizure efficacy, when compared to the racemate of 2-(2-oxopyrrolidin-1-yl)butanamide or to either of the individual enantiomers of 2-(2-oxopyrrolidin-1-yl)butanamide.

All patents, publications, and abstracts cited above are incorporated herein by reference in their entireties. Various embodiments of the invention have been described in fulfillment of the various objectives of the invention. It should be recognized that these embodiments are merely illustrative of the principles of the present invention. Numerous modifications and adaptions thereof will be readily apparent to those skilled in the art without departing from the spirit and scope of the present invention as defined in the following claims.

## Claims

1. A composition comprising a compound of formula (I) and a compound of formula (II),

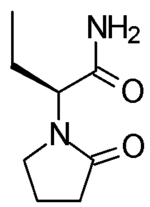

(I)

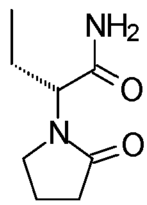

(II)

and/or pharmaceutically acceptable solvates or co-crystals thereof,
wherein the enantiomeric excess (ee) of said compound of formula (I) is equal to or higher than 50% and lower than or equal to 67%.

2. The composition of claim 1, wherein the

compound of formula (I) and/or pharmaceutically acceptable solvates or co-crystals thereof and

compound of formula (II) and/or pharmaceutically acceptable solvates or co-crystals thereof are packaged separately.

**3.** The composition of claim 1, wherein the composition is a non-racemic mixture of 2-(2-oxopyrrolidin-1-yl)butanamide and pharmaceutically acceptable solvates or co-crystals thereof, wherein the non-racemic mixture comprises the compound of formula (I) to the compound of formula (II) in an enantiomeric excess (ee) of the compound of formula (I) of equal to or higher than 50% and lower than or equal to 67%.

**4.** A pharmaceutical composition comprising the composition of any one of the preceding claims and a pharmaceutically acceptable carrier.

**5.** The composition of any one of claims 1 to 3 or the pharmaceutical composition of claim 4 for use as a medicament.

**6.** The composition or the pharmaceutical composition for use of claim 5 in treating and/or preventing a disease, injury or disorder, wherein the disease, injury or disorder is selected from seizure-related disorders.

**7.** The composition for use or the pharmaceutical composition for use of claim 6, wherein the composition or the pharmaceutical composition is to be administered orally twice daily in a dose of between 10 mg and 3000 mg per administration, more preferably between 20 mg to 2000 mg per administration, again more preferably between 50 mg and 1000 mg per administration.

**8.** A method for preparing a composition of any one of claims 1 to 3 or a pharmaceutical composition of claim 4, comprising combining

a compound of formula (I), and a compound of formula (II), or
a compound of formula (I), and a racemate of a compound of formula (I) and (II).

**9.** Use of a compound of formula (I) and/or a compound of formula (II) and/or a racemate of a compound of formula (I) and (II) in the preparation of a composition of any one of claims 1 to 3 or a pharmaceutical composition of claim 4.

## Patentansprüche

**1.** Zusammensetzung, die eine Verbindung der Formel (I) und eine Verbindung der Formel (II)

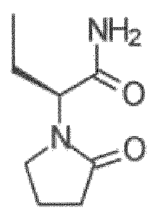

(I)

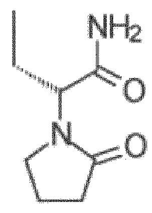

(II)

und/oder pharmazeutisch verträgliche Solvate oder Co-Kristalle davon umfasst,
wobei der Enantiomerenüberschuss (ee) der Verbindung der Formel (I) gleich oder mehr als 50% und weniger als oder gleich 67% ist.

**2.** Zusammensetzung nach Anspruch 1, wobei die Verbindung nach Formel (I) und/oder pharmazeutisch verträgliche Solvate oder Co-Kristalle davon und die Verbindung nach Formel (II) und/oder pharmazeutisch verträgliche Solvate oder Co-Kristalle davon separat verpackt sind.

**3.** Zusammensetzung nach Anspruch 1, wobei die Zusammensetzung ein nichtracemisches Gemisch aus 2-(2-Oxopyrrolidin-1-yl)butanamid und pharmazeutisch verträglichen Solvaten oder Co-Kristallen davon ist, wobei das nicht-racemische Gemisch die Verbindung der Formel (I) im Verhältnis zur Verbindung der Formel (II) in einem Enantiomerenüberschuss (ee) der Verbindung der Formel (I) von gleich oder mehr als 50% und weniger als oder gleich 67% umfasst.

**4.** Pharmazeutische Zusammensetzung, die die Zusammensetzung nach einem der vorangehenden Ansprüche und einen pharmazeutisch verträglichen Träger umfasst.

**5.** Zusammensetzung nach einem der Ansprüche 1 bis 3 oder pharmazeutische Zusammensetzung nach Anspruch 4 zur Verwendung als Medikament.

**6.** Zusammensetzung oder pharmazeutische Zusammensetzung zur Verwendung nach Anspruch 5 zur Behandlung und/oder Vorbeugung einer Erkrankung, Verletzung oder Störung, wobei die Erkrankung, Verletzung oder Störung ausgewählt ist aus mit Anfällen in Zusammenhang stehenden Störungen ausgewählt ist.

**7.** Zusammensetzung zur Verwendung oder pharmazeutische Zusammensetzung zur Verwendung nach Anspruch 6, wobei die Zusammensetzung oder die pharmazeutische Zusammensetzung zweimal täglich in einer Dosis zwischen 10 mg und 3000 mg pro Verabreichung, stärker bevorzugt zwischen 20 mg bis 2000 mg pro Verabreichung, wiederum stärker bevorzugt zwischen 50 mg und 1000 mg pro Verabreichung oral zu verabreichen ist.

**8.** Verfahren zur Herstellung einer Zusammensetzung nach einem der Ansprüche 1 bis 3 oder einer pharmazeutischen Zusammensetzung nach Anspruch 4, umfassend das Kombinieren einer Verbindung der Formel (I) und einer Verbindung der Formel (II) oder einer Verbindung der Formel (I) und eines Racemats einer Verbindung der Formel (I) und (II).

**9.** Verwendung einer Verbindung der Formel (I) und/oder einer Verbindung der Formel (II) und/oder eines Racemats einer Verbindung der Formel (I) und (II) bei der Herstellung einer Zusammensetzung nach einem der Ansprüche 1 bis 3 oder einer pharmazeutischen Zusammensetzung nach Anspruch 4.

## Revendications

**1.** Composition comprenant un composé de formule (I) et un composé de formule (II),

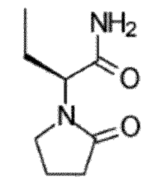
(I)

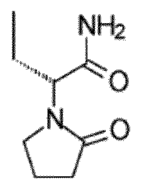
(II)

et/ou solvates ou cocristaux pharmaceutiquement acceptables correspondants,
l'excès énantiomérique (ee) dudit composé de formule (I) étant égal ou supérieur à 50 % et inférieur ou égal à 67 %.

**2.** Composition selon la revendication 1,

le composé de formule (I) et/ou des solvates ou des cocristaux pharmaceutiquement acceptables correspondants et
le composé de formule (II) et/ou des solvates ou des cocristaux pharmaceutiquement acceptables correspondants
étant conditionnés séparément.

**3.** Composition selon la revendication 1, la composition étant un mélange non racémique de 2-(2-oxopyrrolydin-1-yl)butanamide et des solvates ou des cocristaux pharmaceutiquement acceptables correspondants, le mélange non racémique comprenant le composé de formule (I) au composé de formule (II) en un excès énantiomérique (ee)

du composé de formule (I) égal ou supérieur à 50 % et inférieur ou égal à 67 %.

**4.** Composition pharmaceutique comprenant la composition selon l'une quelconque des revendications précédentes et un support pharmaceutiquement acceptable.

**5.** Composition selon l'une quelconque des revendications 1 à 3 ou compositions pharmaceutiques selon la revendication 4 pour une utilisation en tant que médicament.

**6.** Composition ou composition pharmaceutique pour une utilisation selon la revendication 5 dans le traitement et/ou la prévention d'une maladie, d'une lésion ou d'un trouble, la maladie, la lésion ou le trouble étant choisi(e) parmi des troubles liés aux crises épileptiques.

**7.** Composition pour une utilisation ou composition pharmaceutique pour une utilisation selon la revendication 6, la composition ou la composition pharmaceutique devant être administrée oralement deux fois par jour en une dose comprise entre 10 mg et 3 000 mg par administration, plus préférablement entre 20 mg et 2 000 mg par administration, encore plus préférablement entre 50 mg et 1 000 mg par administration.

**8.** Procédé pour la préparation d'une composition selon l'une quelconque des revendications 1 à 3 ou d'une composition pharmaceutique selon la revendication 4, comprenant la combinaison

d'un composé de formule (I), et d'un composé de formule (II), ou
d'un composé de formule (I), et d'un racémique d'un composé de formule (I) et (II).

**9.** Utilisation d'un composé de formule (I) et/ou d'un composé de formule (II) et/ou d'un racémique d'un composé de formule (I) et (II) dans la préparation d'une composition selon l'une quelconque des revendications 1 à 3 ou d'une composition pharmaceutique selon la revendication 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009058261 A1
- EP 0165919 A
- EP 2018064125 W
- US 6570036 B
- US 7544705 B
- US 8334286 B
- US 6784197 B


### Non-patent literature cited in the description


- **FOSTER et al.** *Nature,* 1987, vol. 329, 395-396
- **MAYER et al.** *Trends in Pharmacol. Sci.,* 1990, vol. 11, 254-260
- **PAOLETTI P et al.** *Nat Rev Neurosci.,* 2013, vol. 14 (6), 383-400
- **MARTUCCI L et al.** *Schizophrenia Res,* 2006, vol. 84 (2-3), 214-21
- **LI N et al.** *Biol Psychiatry.,* 2011, vol. 69 (8), 754-61
- **PRESKORN SH et al.** *J Clin Psychopharmacol.,* 2008, vol. 28 (6), 631-7
- **GRIMWOOD S et al.** *Neuroreport.,* 1999, vol. 10 (3), 461-5
- **WEICKERT CS et al.** *Molecular Psychiatry,* 2013, vol. 18, 1185-1192
- **HANSON JE et al.** *Neurobiol Dis.,* 2015, vol. 74, 254-62
- **LI S et al.** *J Neurosci.,* 2011, vol. 31 (18), 6627-38
- **ORGOGOZO JM et al.** *Stroke,* 2002, vol. 33, 1834-1839
- **DUTY S.** *CNS Drugs.,* 2012, vol. 26 (12), 1017-32
- **STEECE-COLLIER K et al.** *Exp Neurol.,* 2000, vol. 163 (1), 239-43
- **LEAVER KR et al.** *Clin Exp Pharmacol Physiol.,* 2008, vol. 35 (11), 1388-94
- **TANG TS et al.** *Proc Natl Acad Sci USA.,* 2005, vol. 102 (7), 2602-7
- **LI L et al.** *J Neurophysiol.,* 2004, vol. 92 (5), 2738-46
- **GRASSELLI G et al.** *Br J Pharmacol.,* 2013, vol. 168 (2), 502-17
- **WANG D et al.** *Expert Opin Ther Targets,* 2014, vol. 18 (10), 1121-30
- **BULLOCK MR et al.** *Ann N YAcad Sci.,* 1999, vol. 890, 51-8
- **YANG Y et al.** *J Neurosurg.,* 2003, vol. 98 (2), 397-403
- **NASPOLINI AP et al.** *Epilepsy Res.,* June 2012, vol. 100 (1-2), 12-9
- **MORISSETTE M et al.** *Mov Disord.,* 2006, vol. 21 (1), 9-17
- **FULLER PI et al.** *Neurosci Lett.,* 2006, vol. 399 (1-2), 157-61
- **NASKAR R et al.** *Semin Ophthalmol.,* September 1999, vol. 14 (3), 152-8
- **WU LJ ; ZHUO M.** *Neurotherapeutics.,* 2009, vol. 6 (4), 693-702
- **PEETERS M et al.** *J Pharmacol Exp Ther.,* 2007, vol. 321 (2), 564-72
- **YUAN H et al.** *Neuron.,* 2015, vol. 85 (6), 1305-18
- **DALMAU J. et al.** *Lancet Neurol.,* 2008, vol. 7 (12), 1091-8
- **WON H. et al.** *Nature,* 2012, vol. 486 (7402), 261-5
- **TANG, Y. P. et al.** *Nature,* 1999, vol. 401 (6748), 63-9
- **ARNOLD PD et al.** *Psychiatry Res.,* 2009, vol. 172 (2), 136-9
- **DORVAL KM et al.** *Genes Brain Behav.,* 2007, vol. 6 (5), 444-52
- **HALLER J et al.** *Behav Pharmacol.,* 2011, vol. 22 (2), 113-21
- **LEADERBRAND K et al.** *Neurobiol Learn Mem.,* 2014, vol. 113, 35-40
- **GUITTON MJ ; DUDAI Y.** *Neural Plast.,* 2007, 80904
- **STRAUBE A. et al.** *Curr Opin Neurol.,* 2005, vol. 18 (1), 11-4
- **STARCK M et al.** *J Neurol.,* January 1997, vol. 244 (1), 9-16
- **KOWAL C et al.** *Proc. Natl. Acad. Sci. U.S.A.,* 2006, vol. 103, 19854-19859
- **NAGY J.** *Curr Drug Targets CNS Neurol Disord.,* 2004, vol. 3 (3), 169-79
- **SHEN H et al.** *Proc Natl Acad Sci USA.,* 2011, vol. 108 (48), 19407-12

- **SINGH JB et al.** *Biol Psychiatry,* 2016, vol. 80 (6), 424-431
- **PRESKORN SH et al.** *J Clin Psychopharmacol,* 2008, vol. 28 (6), 631-7
- **RUSH et al.** *Am. J. Psychiatry,* 2006, vol. 163, 1905
- **SARA et al.** *Psychopharmacology (Berl).,* 1980, vol. 68 (3), 235-41
- **VAN AKEN et al.** *Acta Anaesthesiol Belg.,* 1980, vol. 31 (21-8
- **GOWER et al.** *Eur J Pharmacol.,* 10 November 1992, vol. 222 (2-3), 193-203
- **VERLOES R et al.** Effects of nootropic drugs in a scopolamine-induced amnesia model in mice. *Psychopharmacology (Berl).,* 1988, vol. 95 (2), 226-30
- **LYNCH et al.** *Proc Natl Acad Sci USA.,* 29 June 2004, vol. 101 (26), 9861-6
- **CARUNCHIO et al.** *Epilepsia,* April 2007, vol. 48 (4), 654-62
- **FARIELLO RG et al.** *Neuropharmacology.,* 2014, vol. 81, 85-94
- **PINZA M et al.** *J Med Chem,* 1993, vol. 36 (26), 4214-20
- Remington: The Science and Practice of Pharmacy. Pharmaceutical Press
- RSC Drug Discovery, Pharmaceutical Salts and Co-crystals. the Royal Society of Chemistry, 2012
- **ATTAL N ; CRUCCU G ; BARON R ; HAANPÄÄ M ; HANSSON P ; JENSEN TS ; NURMIKKO T.** European Federation of Neurological Societies. EFNS guidelines on the pharmacological treatment of neuropathic pain: 2010 revision. *Eur J Neurol.,* September 2010, vol. 17 (9), 1113-e88
- **BRADFORD MM.** A rapid and sensitive method for the quantitation of microgram quantities of protein utilizing the principle of protein-dye binding. *Anal Biochem.,* 07 May 1976, vol. 72, 248-54
- **BRIL V ; ENGLAND J ; FRANKLIN GM ; BACKONJA M ; COHEN J ; DEL TORO D ; FELDMAN E ; IVERSON DJ ; PERKINS B ; RUSSELL JW.** American Academy of Neurology; American Association of Neuromuscular and Electrodiagnostic Medicine; American Academy of Physical Medicine and Rehabilitation. Evidence-based guideline: Treatment of painful diabetic neuropathy: report of the American Academy of Neurology, the American Association of Neuromuscular and Electrodiagnostic Medicine, and the American Academy of Physical Medicine and Rehabilitation. *Neurology.,* 17 May 2011, vol. 76 (20), 1758-65
- *Neurology.,* 09 August 2011, vol. 77 (6), 603
- **CAMILLERI P ; EGGLESTON D ; FARINA C ; MURPHY JA ; PFEIFFER U ; PINZA M ; SENIOR LA.** Chiral high-performance liquid chromatography of some related bicyclic lactams. *Journal of Chromatography A,* 1993, vol. 654, 207-213
- **CAVALETTI G ; TREDICI G ; PETRUCCIOLI MG ; DONDÈ E ; TREDICI P ; MARMIROLI P ; MINOIA C ; RONCHI A ; BAYSSAS M ; ETIENNE GG.** Effects of different schedules of oxaliplatin treatment on the peripheral nervous system of the rat. *Eur J Cancer.,* December 2001, vol. 37 (18), 2457-63
- **CHRISTENSEN D ; IDÄNPÄÄN-HEIKKILÄ JJ ; GUILBAUD G ; KAYSER V.** The antinociceptive effect of combined systemic administration of morphine and the glycine/NMDA receptor antagonist, (+)-HA966 in a rat model of peripheral neuropathy. *Br J Pharmacol.,* December 1998, vol. 125 (8), 1641-50
- *Br J Pharmacol,* April 1999, vol. 126 (8), 1881
- **DUBINSKY RM ; KABBANI H ; EL-CHAMI Z ; BOUTWELL C ; ALI H.** Quality Standards Subcommittee of the American Academy of Neurology. Practice parameter: treatment of postherpetic neuralgia: an evidence-based report of the Quality Standards Subcommittee of the American Academy of Neurology. *Neurology.,* 28 September 2004, vol. 63 (6), 959-65
- **DI CESARE MANNELLI L ; MARESCA M ; FARINA C ; SCHERZ MW ; GHELARDINI C.** A model of neuropathic pain induced by sorafenib in the rat: Effect of unifiram. *Neurotoxicology,* September 2015, vol. 50, 101-7
- **DI CESARE MANNELLI L ; MICHELI L ; FARINA C ; SCHERZ M ; GHELARDINI C.** Effects of unifiram on oxaliplatin-induced hyperalgesia and allodynia in the rat. *Journal of Clinical Oncology,* 2015, vol. 33 (15), e20650-e20650
- **LIEL EL ; WILEN SH.** Stereochemistry of Organic Compounds. Wiley-Interscience, 1994
- **FARINA C ; GAGLIARDI S ; GHELARDINI C ; MARTINELLI M ; NORCINI M ; PARINI C ; PETRILLO P ; RONZONI S.** *Bioorg Med Chem.,* 2008, vol. 16 (6), 3224-32
- **FARIELLO RG ; GHELARDINI C ; DI CESARE MANNELLI L ; BONANNO G ; PITTALUGA A ; MILANESE M ; MISIANO P ; FARINA C.** Broad spectrum and prolonged efficacy of unifiram in models of neuropathic pain. *Neuropharmacology,* June 2014, vol. 81, 85-94
- Antidepressant-like activity of unifiram (NT-11624) in the rat forced swimming test. Program No. 789.08/EE25. **FARIELLO RG ; GHELARDINI C ; DI CESARE MANNELI L ; ZANARDELLI M ; FARINA C.** 2011 Neuroscience Meeting Planner. Society for Neuroscience, 2011
- **FERNIHOUGH J ; GENTRY C ; MALCANGIO M ; FOX A ; REDISKE J ; PELLAS T ; KIDD B ; BEVAN S ; WINTER J.** Pain related behaviour in two models of osteoarthritis in the rat knee. *Pain,* November 2004, vol. 112 (1-2), 83-93

- **FINNERUP NB ; ATTAL N ; HAROUTOUNIAN S ; MCNICOL E ; BARON R ; DWORKIN RH ; GILRON I ; HAANPÄÄ M ; HANSSON P ; JENSEN TS.** Pharmacotherapy for neuropathic pain in adults: a systematic review and meta-analysis. *Lancet Neurol.,* February 2015, vol. 14 (2), 162-73
- **GRONSETH G ; CRUCCU G ; ALKSNE J ; ARGOFF C ; BRAININ M ; BURCHIEL K ; NURMIKKO T ; ZAKRZEWSKA JM.** Practice parameter: the diagnostic evaluation and treatment of trigeminal neuralgia (an evidence-based review): report of the Quality Standards Subcommittee of the American Academy of Neurology and the European Federation of Neurological Societies. *Neurology,* 07 October 2008, vol. 71 (15), 1183-90
- **GUINGAMP C ; GEGOUT-POTTIE P ; PHILIPPE L ; TERLAIN B ; NETTER P ; GILLET P.** Mono-iodoacetate-induced experimental osteoarthritis: a dose-response study of loss of mobility, morphology, and biochemistry. *Arthritis Rheum.,* September 1997, vol. 40 (9), 1670-9
- **GUZMAN RE ; EVANS MG ; BOVE S ; MORENKO B ; KILGORE K.** Mono-iodoacetate-induced histologic changes in subchondral bone and articular cartilage of rat femorotibial joints: an animal model of osteoarthritis. *Toxicol Pathol.,* 2003, vol. 31 (6), 619-24
- **LATREMOLIERE A ; WOOLF CJ.** Central sensitization: a generator of pain hypersensitivity by central neural plasticity. *J Pain.,* September 2009, vol. 10 (9), 895-926
- **HANSEN KB ; YI F ; PERSZYK E ; FURUKAWA H ; WOLLMUTH LP ; GIBB AJ ; TRAYNELIS SF.** Structure, function, and allosteric modulation of NMDA receptors. *J Gen Physiol,* 2018, vol. 150 (8), 1081-1105
- **MANETTI D ; GHELARDINI C ; BARTOLINI et al.** Design, synthesis and preliminary pharmacological evaluation of 1,4-diazabicyclo[4.3.0]nonan-9-ones as a new class of highly potent nootropic drugs. *J Med Chem,* 2000, vol. 43, 1969-1974
- **MARTINI E ; GHELARDINI C ; BERTUCCI C et al.** Enantioselective synthesis and preliminary pharmacological evaluation of the enantiomers of unifiram (DM232), a potent cognition-enhancing agent. *Med Chem,* 2005, vol. 1, 473-480
- Human Anatomy. Pearson, 2017
- **MOULIN D ; BOULANGER A ; CLARK AJ ; CLARKE H ; DAO T ; FINLEY GA ; FURLAN A ; GILRON I ; GORDON A ; MORLEY-FORSTER PK.** Canadian Pain Society. Pharmacological management of chronic neuropathic pain: revised consensus statement from the Canadian Pain Society. *Pain Res Manag.,* November 2014, vol. 19 (6), 328-35
- **NAKAMURA Y ; IGA K ; SHIBATA T ; SHUDO M ; KATAOKA K.** Glial plasmalemmal vesicles: a subcellular fraction from rat hippocampal homogenate distinct from synaptosomes. *Glia,* September 1993, vol. 9 (1), 48-56
- **PALUZZI S ; ALLOISIO S ; ZAPPETTINI S ; MILANESE M ; RAITERI L ; NOBILE M ; BONANNO G.** Adult astroglia is competent for Na+/Ca2+ exchanger-operated exocytotic glutamate release triggered by mild depolarization. *J Neurochem,* November 2007, vol. 103 (3), 1196-207
- **PINZA M ; FARINA C ; CERRI A ; PFEIFFER U ; RICCABONI MT ; BANFI S ; BIAGETTI R ; POZZI O ; MAGNANI M ; DORIGOTTI L.** Synthesis and pharmacological activity of a series of dihydro-1H-pyrrolo[1,2-a]imidazole-2,5(3H,6H)-diones, a novel class of potent cognition enhancers. *J Med Chem.,* 24 December 1993, vol. 36 (26), 4214-20
- **RANDALL LO ; SELITO JJ.** A method for measurement of analgesic activity on inflamed tissue. *Arch Int Pharmacodyn Ther.,* 01 September 1957, vol. 111 (4), 409-19
- **SCAPECCHI S ; MARTINI E ; MANETTI D et al.** Structure-activity relationship studies on unifiram (DM232) and sunifiram (DM235), two novel and potent cognition enhancing drugs. *Bioorg Med Chem,* 2004, vol. 12, 71-85
- **SMITH BH ; TORRANCE N ; BENNETT MI ; LEE AJ.** Health and quality of life associated with chronic pain of predominantly neuropathic origin in the community. *Clin J Pain.,* February 2007, vol. 23 (2), 143-9
- **SPRINGUEL GR ; LEYSSENS T.** Innovative chiral resolution using enantiospecific co-crystallization in solution. *Cryst Growth Des.,* 2012, vol. 12 (7), 3374-3378
- **TORCHIO L ; LOMBARDI F ; VISCONTI M ; DOYLE E.** Determination of the polar drug unifiram in human plasma and serum by column-switching high-performance liquid chromatography. *J Chromatogr B Biomed Appl.,* 07 April 1995, vol. 666 (1), 169-77
- **VAN HECKE O ; AUSTIN SK ; KHAN RA ; SMITH BH ; TORRANCE N.** Neuropathic pain in the general population: a systematic review of epidemiological studies. *Pain,* April 2014, vol. 155 (4), 654-62
- *Pain.,* September 2014, vol. 155 (9), 1907
- Crystallization-based separation of enantiomers. **WANG Y ; CHEN A.** Stereoselective Synthesis of Drugs and Natural Products. Wiley-Interscience, 2013, vol. 2
- **ZILLIOX LA.** Neuropathic Pain. *Continuum (Minneap Minn).,* April 2017, vol. 23 (2), 512-532
- **GOWER et al.** *Eur. J. Pharmacol.,* 1992, vol. 222, 193-203
- **LOSCHER W et al.** *J. Pharm. Exp. Ther,* 1998, vol. 284, 474-479